# EUROPEAN PATENT APPLICATION

(11) **EP 1 444 959 A1**
(43) Date of publication of application: **11.08.2004**
(21) Application number: 03075368.5
(22) Date of filing: 07.02.2003
(51) Int. Cl.: A61B 17/82

(54) **Bone fixing device**

(71) Applicant: DSM IP Assets B.V., 6411 TE Heerlen (NL)
(72) Inventor: Dirks, Christiaan Henri Peter, 3650 Dilsen (BE); Marissen, Roelof, 6121 HS Born (NL)
(74) Representative: Krijgsman, Willem

(57) **Abstract**

Closed loop of high performance polyethylene fibers for use as a bone-fixing tool, and method for fixing bone parts by a surgical cable comprising the steps of laying the surgical cable, having two end parts, around at least part of the bone parts to be fixed, exerting a force on the end parts bringing the cable under a tension required for the fixing of the bone parts and locking the tensioned cable against the influence of forces acting counter to the exerted force.

## Description

The invention relates to a method for fixing bone parts by means of a surgical cable.

In modern surgery on many occasions there is a need for internally immobilizing bone parts that have been separated in the course of an operation and have to grow together again or to keep a bone part at a fixed and constant distance and position with respect to another bone part or an orthopedic device such as a splint, further denoted as fixing bone parts. Also in the treatment of bone fractures this need for fixing bone parts that came apart is required for at least the time needed to have the body repair the fracture or for longer times, in many cases even for years.

It is known in surgery to wrap a steel cable around the bone parts to be fixed, to bring the cable under the required tension to fix the parts again relative movement, e.g. under load, and to leave it in place inside the body at least until the bone parts have grown together and the bone has recovered sufficiency to take up its proper function again, or even permanently to avoid a further operation to remove the cable. The cable is tensioned and fixed by guiding its ends from opposite sides through holes in a metal block, tensioning the cable by exerting a drawing force on the ends and pinching the metal block such that the holes collapse and fix the cable.

The use of steel cables brings about a number of disadvantages. They are prone to fatigue leading to breakage of the composing steel fibres after which the sharp ends stick out into the body. Breakage of the fibers during their application by a surgeon brings the risk of stitching and possible blood contact. Further, steel is a hard material and being tensioned around the bone there is the risk of carving of the steel cable into the bone.

US Patent 5,540,703 teaches to use instead of a metal cable a braided polymeric material cable and to lock the tightened and tensioned cable with non-loosening knots, in order to overcome certain disadvantages of metal cables. High performance, i.e. high strength, high modulus, polyethylene in particular is applied as the polymeric material. Fibers of this type, however, are notorious for their difficulty to be fixed by knots, clamps or other means when they are under tension.

The present invention now seeks to provide a method and means for fixing bone parts by means of a surgical cable of a polymeric material that do not suffer from the disadvantages of the known means and cope with the fixing difficulties related to the application of tensioned high performance fibers.

The invention thus relates to a method for fixing bone parts comprising the steps of laying a surgical cable having two end parts, around at least part of the bone parts to be fixed, exerting a force on the end parts bringing the cable under a tension required for the fixing of the bone parts and locking the tensioned cable against the influence of forces acting counter to the exerted force.

In the method according to the invention a fiber surgical cable having two ends is applied. The fiber is a high performance fiber, preferably a polyethylene fiber having a tensile strength of at least 1.8 GPa and a modulus of at least 60 GPa. Examples of such fibers are various Dyneema grades of DSM High Performance Fibers and various Spectra grades of Honeywell Inc. These fibers have been prepared from high molecular weight polyethylene, in particular polyethylene heaving a weight average molecular weight of at least 2,000,000.

In particular the cable is a bundle of parallel, twisted or braided fibers of the type described above. It may also be a high performance tape having the required strength and modulus. The tape may be a single tape or it may be in the form of a flat braid of high performance fibers. Twisting and braiding are commonly applied techniques in cable production and cables obtained by these common techniques are applicable in the device according to the invention. It should be noted that in constructions of these fibers, e.g. in braids and twisted bundles an efficiency loss occurs, i.e. that the resulting strength of the construction is lower that then the sum of the strengths of the constituting fibers. The efficiency depends on the used braid construction, braiding period and braiders. Braid efficiency may range from 30-70%. Starting from the required strength in each case the proper combination of initial fiber strength, cable thickness and cable construction can be chosen to obtain a cable having at least that required strength. The forces required to fix bone parts generally range from 500 to 3000 N, depending on the size of the bones to be fixed and the forces exerted on the bone parts. For small bones, like in fingers, smaller forces and thicknesses may be relevant. In general the total thickness of the cable will range from 500 to 30,000 dtex.

The cable must be suited to be positioned around the bone parts and has an oblong shape; in particular the cable is a bundle of parallel, twisted or braided fibers of a length that is sufficient to be laid around the bone parts to be fixed and to be tensioned.

The cable in the described shape of a bundle of fibers has two ends. These ends normally will have been treated to prevent unraveling or splitting of the bundle. The ends can e.g. have been treated with a substance gluing together the fibers, have been molten together or otherwise be prevented from unraveling. In this embodiment the last few centimeters of the bundle to the ends form the end parts. In another embodiment a fiber end may have been formed into an eye by splicing the end back into the bundle. In this case with the end parts of the fiber the eyes are meant.

In another embodiment the ends of a bundle of fibers of finite length may have been spliced together, in particular by air splicing, to form an endless loop. The fibers may be parallel or twisted over at least part of their length. In this embodiment the cable will be applied by flattening the loop to give an oblong object and the end parts in this case are understood to be the returning ends of the flattened loop. This closed loop can also be given a half-twist and be folded together to form again a closed loop, now of half the diameter of the original loop and twice its thickness. Depending on the length of the original bundle of fibers and the thickness required this procedure can be repeated.

The cable is positioned around the bone parts following a trajectory that is stable when the cable is tensioned. This prevents the cable from moving to a shorter trajectory, leading to loss of tension in the cable and consequently loss of fixing. Generally this will be the shortest trajectory at a certain position along the bone parts. Alternatively the cable along the trajectory may be prevented from sliding to a shorter one by natural obstacles as bone processes or artificial fixing devices or protrusions applied to the bone parts.

A force is exerted on the end parts to bring the cable under a tension required for the fixing of the bone parts. If a bundle of finite length is applied, preferably the end parts are knotted together. Suitable knots for this purpose are in principle elementary simple knots since, in the method of the invention, they will have to withstand much smaller forces since they will not be directly loaded under tension. Examples of suitable knots are a loop knot, a water knot, tape knot, a double figure eight knot and a double overhand bend. With good results a double figure eight knot and a double overhand bend is applied as a first knot, and preferably a further knot, e.g. two flat half or flat overhand knots, is applied on top of the first knot. A force is then exerted to tension the cable around the bone parts. Preferably a force bringing about torsion is exerted, e.g. by connecting a device, e.g. a stick shaped object to the knot in the end parts and turning it around to twist the end parts of the cable. The two parts of the cable extending from the knot facing the bone parts and forming a loop around the bone parts thus will be twisted resulting in shortening and tensioning of the cable. It is clear that most tension built up diminishes along the twisted part towards the knot. Consequently the knot is only lightly loaded and is not critical indeed.

In a preferred embodiment of the invention a turning device is shoved between the bone part closest to the knot and the knot and turned around in a plane parallel to the bone part. Also in this way part of the cable is twisted resulting in shortening and tensioning of the cable. More preferably the device is hooked or otherwise connected to the cable and can be e.g. stick-shaped and having a bend or an eye that fits around the fiber bundle of the cable. This promotes the fixing of the object to the twisted cable preventing slipping of the device out of between the twists in the cable.

If a cable in the form of a flattened closed loop is applied the flattened loop can be longer than the circumference of the trajectory around the bone parts to be connected or it can be shorter.

In the former case, in analogy to the embodiment described here above, a device can be shoved through the eyes formed by the returning end parts and as described above by twisting the device the cable will be tensioned. Also in this case the device preferably is connected to the cable to avoid slipping out of the device.

In the latter case also a turning device can be shoved through the eyes formed by the returning end parts but in this case this device has to bridge the gap between the two end parts and run over part of a bone part. Preferably in this case the turning device is a second fiber bundle in one of the shapes as described here above for the cable. On this second fiber bundle then a force, preferably a twisting force, is exerted in order to shorten and tension this bundle. This shortening then leads to a tensioning force on the cable tending to close the gap between the eyes of the end parts of the cable.

Another way of tensioning the cable in said latter case is the application of a device in the gap between the eyes forming the end parts of the cable that exerts a drawing force rather than a twisting force. Such a device should be adapted for holding the end parts of the cable. In case a cable having end parts in the form of an eye is applied the device may comprise two hooks or similar that each can hook to one of the eyes of the cable and be provided with means to draw the hooks to one another. Such means can comprise a mechanism as used in turn buckle, a worm wheel and driving screw combination or two cooperating 45 ° tooth wheels rotating around mutually perpendicular axes. These tensioning devices can be connected to the cable in such a way that only a drawing force is exerted on the cable, resulting in its shortening and tensioning but also in such a way that, instead of or next to the drawing force, also a twisting force is exerted the cable, also resulting in further tensioning the cable.

In all embodiments disclosed the tensioning action is continued until the required tension in the cable is achieved. Subsequently the tensioned cable must be locked against the influence of forces acting counter to the exerted force. When a drawing force has been exerted this can be done by fixing the rotating part of the device against rotating back. Means for such fixing are known per se and can be used in the present invention. When a twisting force has been exerted to tension the cable then a hook or an eye present on the turning device can be hooked to the cable or to a corresponding protrusion, hook, eye or clamp connected to one of the bone parts. It should be noted that the force required to prevent the untwisting of the cable is much less than the tension force in the cable. This implies that the problem of clamping or knotting high performance polyethylene fibers of the prior art is absent in the method according to the invention.

The application of a rotating force in this respect has as large advantage that the tensioning effect of it can be much larger than the rotating force itself. In the embodiments described above in which a separate object is applied to exert the rotating force it generally is sufficient to connect one end, in particular the end that is not connected to the end part of the cable, to the fibers of the cable, e.g. by hooking the object to the fibers. The object exerts virtually no tensioning force but mainly a rotating force that can easily be absorbed by the fiber bundle.

The various embodiment described methods are not only suitable for the fixing of bone parts but they are also useful for the connection of bones to artificial elements providing some orthotic function, e.g. a splint.

## Claims

1. Method for fixing bone parts by a surgical cable comprising the steps of laying the surgical cable, having two end parts, around at least part of the bone parts to be fixed, exerting a force on the end parts bringing the cable under a tension required for the fixing of the bone parts and locking the tensioned cable against the influence of forces acting counter to the exerted force.

2. Method according to claim 1, wherein the polymer fiber is a high performance high molecular weight fiber.

3. Method according to Claim 1 or 2, wherein the exerted force is a torsion force.

4. Method according to one of the claims 1-3 wherein the cable is twisted yarn having an eye at least at one of the end parts.

5. Method according to claim 4 wherein the cable has an eye at both ends

6. Method according to claim 4 or 5 wherein the torsion force is exerted on the cable through the eye or the eyes.

7. Method according to claim 5 wherein the torsion force is exerted on a twisting device running through the eyes.

8. Method according to claim 1 or 2, wherein the fiber cable is a loop of fibers that has been closed by a splice, preferably an air splice, which is folded around the bone parts forming two returning ends in the cable as end parts.

9. Method according to claim 8 wherein the torsion force is exerted on the cable through the returning ends.

10. Method according to claim 9, wherein the torsion force is exerted on a twisting device running through the returning ends.

11. Method according to claims 1 or 2, wherein the cable is fiber bundle of finite length.

12. Method according to claim 8 or 11, wherein the two end parts are connected with a knot.

13. Method according claim 12, wherein a torsion force is exerted on the cable below the knot

14. Closed loop of high performance polyethylene fibers for use as a bone-fixing tool.
